(19) Europäisches Patentamt
European Patent Office
Office européen des brevets

(11) EP 4 261 248 A1

(12) **EUROPEAN PATENT APPLICATION**

(43) Date of publication:
**18.10.2023 Bulletin 2023/42**

(21) Application number: **23167632.1**

(22) Date of filing: **12.04.2023**

(51) International Patent Classification (IPC):
***C08K 5/20*** *(2006.01)*

(52) Cooperative Patent Classification (CPC):
(C-Sets available)
**C08K 5/20;** C08K 5/0016 (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **12.04.2022 TW 111113899**

(71) Applicant: **Largan Medical Co., Ltd.**
**Taichung City 408 (TW)**

(72) Inventors:
- **Chen, Wei-Yuan**
  **408 Taichung City (TW)**
- **Chen, Po-Tsun**
  **408 Taichung City (TW)**
- **Lu, Tzu-Rong**
  **408 Taichung City (TW)**
- **Chen, Rih-Sian**
  **408 Taichung City (TW)**
- **Hong, Yu Jie**
  **408 Taichung City (TW)**
- **Teng, Chun-Hung**
  **408 Taichung City (TW)**

(74) Representative: **Lang, Christian**
**LangPatent Anwaltskanzlei IP Law Firm**
**Ingolstädter Straße 5**
**80807 München (DE)**

# (54) PLASTIC COMPOSITION, PLASTICIZER, AND PLASTIC PRODUCT

(57) A plastic composition includes at least one plastic and at least one plasticizer. The plastic includes a polylactic acid. The plasticizer includes an amide ester compound, and the amide ester compound has the biodegradability. The weight percentage of the plastic in the plastic composition is 85% to 99.99%, and the weight percentage of the plasticizer in the plastic composition is 0.01% to 15%.

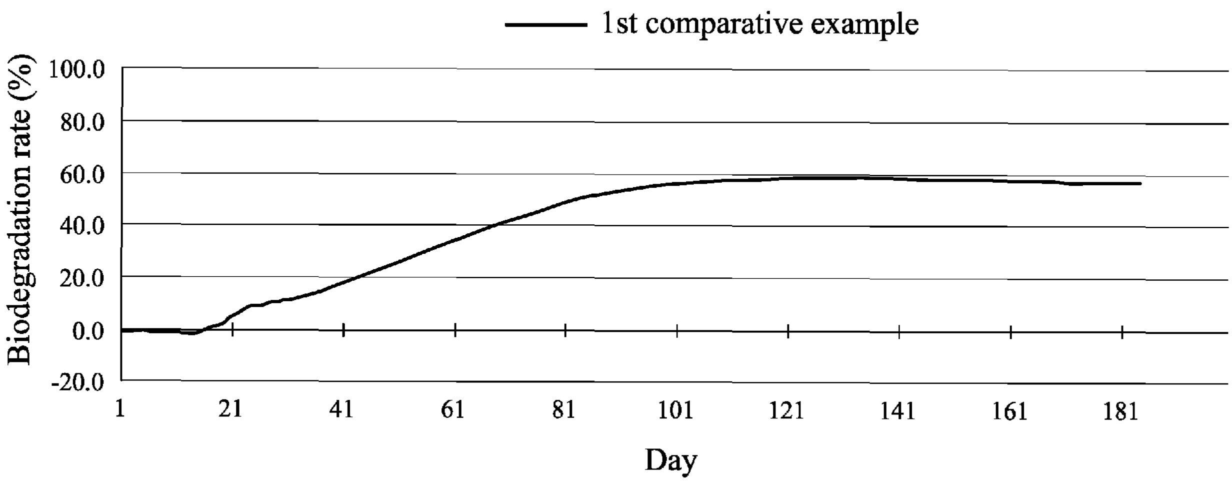


Fig. 1

EP 4 261 248 A1

(52) Cooperative Patent Classification (CPC): (Cont.)

C-Sets
**C08K 5/20, C08L 67/04**

## Description

### BACKGROUND

Technical Field

The present disclosure relates to a plastic composition, a plasticizer, and a plastic product. More particularly, the present disclosure relates to a plastic composition, a plasticizer and a plastic product that have a biodegradability.

Description of Related Art

The plasticizer is a material used to increase the plasticity of the product, and is often used in the plastic product, such as plasticization of the plastic bags. The commonly used traditional plasticizers are mostly phthalate compounds, such as DEHP, DBP, DINP, BBP and DIDP, etc. The plasticizer structure is similar to estrogen, which is easy to cause the abnormal development of adolescent children, and even increase the incidence of breast cancer and ovarian cancer. The molecular weights of the abovementioned traditional plasticizers are mostly less than 500 g/mole, and the smaller molecular weights are relatively easy to be absorbed by the human body. Since the plasticizers are combined with other substances by non-chemical bonding method in the plastic products, which are easily released from the plastic products. Therefore, the plasticizers will be absorbed by the human body when the plastic products contact with food or skin.

In the recent years, the environmental protection awareness has risen, and if the traditional plastic products are not properly treated, they will damage to the environment and the ecology, so that more and more biodegradable products are widely promoted, but the biodegradation rate is often not high. Most plasticizers and plastics still cannot be decomposed, and the users will misunderstand that these products will not affect the environment and the ecology by any disposal due to these products are biodegradable, resulting in these products not being properly treated and further damage the environment and the ecology. Nowadays, the plastic products have become an indispensable part of human life, so that it is very important to find the plasticizers that are not easily released from the plastic products or absorbed by the human body, and to develop the biodegradable plastic products.

### SUMMARY

According to one aspect of the present disclosure, a plastic composition includes at least one plastic and at least one plasticizer. The plastic includes a polylactic acid. The plasticizer includes an amide ester compound, and the amide ester compound has a biodegradability. A weight percentage of the plastic in the plastic composition is 85% to 99.99%, and a weight percentage of the plasticizer in the plastic composition is 0.01% to 15%.

According to the plastic composition of the foregoing aspect, a biodegradation rate on a 14th day is Dr14, and the following condition of the biodegradation rate of the plastic composition on the 14th day can be satisfied: $1\% \leq Dr14$.

According to the plastic composition of the foregoing aspect, a biodegradation rate on a 21th day is Dr21, and the following condition of the biodegradation rate of the plastic composition on the 21th day can be satisfied: $6\% \leq Dr21$.

According to the plastic composition of the foregoing aspect, a biodegradation rate on a 28th day is Dr28, and the following condition of the biodegradation rate of the plastic composition on the 28th day can be satisfied: $15\% \leq Dr28$.

According to the plastic composition of the foregoing aspect, a biodegradation rate on a 42th day is Dr42, and the following condition of the biodegradation rate of the plastic composition on the 42th day can be satisfied: $20\% \leq Dr42$.

According to the plastic composition of the foregoing aspect can further include at least one non-biodegradable plastic, wherein a weight percentage of the at least one non-biodegradable plastic in the plastic composition is less than a weight percentage of the polylactic acid in the plastic composition.

According to the plastic composition of the foregoing aspect, the plasticizer has a structure represented by formula (I):

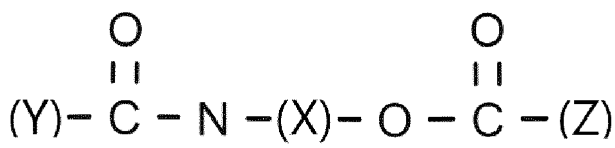

formula (I),

wherein X is a central structure, Y is a first long-chain structure, Z is a second long-chain structure, a number of carbon

atoms in X is XC, a number of carbon atoms in Y is YC, a number of carbon atoms in Z is ZC, and the following conditions can be satisfied: 1 ≤ XC ≤ 6; 14 ≤ YC ≤ 25; and 14 ≤ ZC ≤ 25.

According to the plastic composition of the foregoing aspect, a molecular weight of the plasticizer is Mw, and the following condition can be satisfied: 500 g/mole ≤ Mw.

According to the plastic composition of the foregoing aspect, each of X, Y and Z can be a straight-chain structure.

According to the plastic composition of the foregoing aspect, each of Y and Z can have at least one double bond.

According to the plastic composition of the foregoing aspect, a pyrolysis temperature of the plasticizer is Pt, and the following condition can be satisfied: 100°C ≤ Pt ≤ 300°C.

According to another aspect of the present disclosure, a plastic product includes the plastic composition according to the foregoing aspect.

According to further another aspect of the present disclosure, a plastic composition includes at least one plastic and at least one plasticizer. The plasticizer has a biodegradability, and the plasticizer has a structure represented by formula (I):

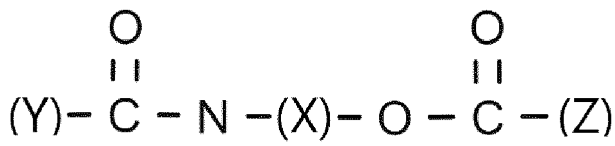

formula (I),

wherein X is a central structure, Y is a first long-chain structure, Z is a second long-chain structure, and when a number of carbon atoms in X is XC, a number of carbon atoms in Y is YC, and a number of carbon atoms in Z is ZC, the following conditions are satisfied: 1 ≤ XC ≤ 6; 14 ≤ YC ≤ 25; and 14 ≤ ZC ≤ 25.

According to the plastic composition of the foregoing aspect, a molecular weight of the plasticizer is Mw, and the following condition can be satisfied: 500 g/mole ≤ Mw.

According to the plastic composition of the foregoing aspect, a weight percentage of the plastic in the plastic composition can be 85% to 99.99%, and a weight percentage of the plasticizer in the plastic composition can be 0.01% to 15%.

According to the plastic composition of the foregoing aspect, each of X, Y and Z can be a straight-chain structure.

According to the plastic composition of the foregoing aspect, each of Y and Z can have at least one double bond.

According to the plastic composition of the foregoing aspect, a pyrolysis temperature of the plasticizer is Pt, and the following condition can be satisfied: 100°C ≤ Pt ≤ 300°C.

According to the plastic composition of the foregoing aspect, a biodegradation rate on a 14th day is Dr14, and the following condition of the biodegradation rate of the plastic composition on the 14th day can be satisfied: 1% ≤ Dr14.

According to the plastic composition of the foregoing aspect, a biodegradation rate on a 21th day is Dr21, and the following condition of the biodegradation rate of the plastic composition on the 21th day can be satisfied: 6% ≤ Dr21.

According to the plastic composition of the foregoing aspect, a biodegradation rate on a 28th day is Dr28, and the following condition of the biodegradation rate of the plastic composition on the 28th day can be satisfied: 15% ≤ Dr28.

According to the plastic composition of the foregoing aspect, a biodegradation rate on a 42th day is Dr42, and the following condition of the biodegradation rate of the plastic composition on the 42th day can be satisfied: 20% ≤ Dr42.

According to still another aspect of the present disclosure, a plastic product includes the plastic composition according to the foregoing aspect.

According to yet another aspect of the present disclosure, a plasticizer has a biodegradability, and the plasticizer has a structure represented by formula (I):

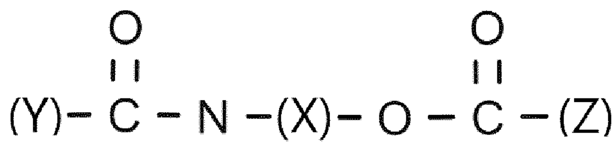

formula (I),

wherein X is a central structure, Y is a first long-chain structure, Z is a second long-chain structure, and when a number

of carbon atoms in X is XC, a number of carbon atoms in Y is YC, and a number of carbon atoms in Z is ZC, the following conditions are satisfied: 1 ≤ XC ≤ 6; 14 ≤ YC ≤ 25; and 14 ≤ ZC ≤ 25. When a biodegradation rate on a 7th day is Dr7, the following condition of the biodegradation rate of the plasticizer on the 7th day is satisfied: 20% ≤ Dr7.

According to the plasticizer of the foregoing aspect, a molecular weight of the plasticizer is Mw, and the following condition can be satisfied: 500 g/mole ≤ Mw.

According to the plasticizer of the foregoing aspect, a biodegradation rate on a 21th day is Dr21, and the following condition of the biodegradation rate of the plasticizer on the 21th day can be satisfied: 65% ≤ Dr21.

According to the plasticizer of the foregoing aspect, each of X, Y and Z can be a straight-chain structure.

According to the plasticizer of the foregoing aspect, each of Y and Z can have at least one double bond.

According to the plasticizer of the foregoing aspect, a pyrolysis temperature of the plasticizer is Pt, and the following condition can be satisfied: 100°C ≤ Pt ≤ 300°C.

According to more another aspect of the present disclosure, a plastic product includes the plasticizer according to the foregoing aspect.

## BRIEF DESCRIPTION OF THE DRAWINGS

The present disclosure can be more fully understood by reading the following detailed description of the embodiment, with reference made to the accompanying drawings as follows:

Fig. 1 is a graph between the biodegradation rate and the number of days of the plastic composition of the 1st comparative example.

Fig. 2 is a graph between the biodegradation rate and the number of days of the plastic composition of the 2nd comparative example.

Fig. 3 is a graph between the biodegradation rate and the number of days of the plastic composition of the 3rd comparative example.

Fig. 4 is a graph between the biodegradation rate and the number of days of the plastic composition of the 1st example.

Fig. 5 is a graph between the biodegradation rate and the number of days of the plastic composition of the 2nd example.

## DETAILED DESCRIPTION

A plastic composition includes at least one plastic and at least one plasticizer. The plastic includes a polylactic acid. The plasticizer includes an amide ester compound, and the amide ester compound has a biodegradability. Therefore, the damage to the environment and the ecology can be reduced by the characteristics of biodegradable polylactic acid and plasticizer. Further, the plasticizer can be prevented from affecting the normal physiological functions by designing the plasticizer structure that is dissimilar to estrogen.

A plastic composition includes at least one plastic and at least one plasticizer. The plasticizer has a biodegradability, and has a structure represented by formula (I):

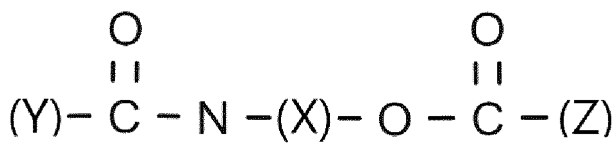

formula (I),

wherein X is a central structure, Y is a first long-chain structure, Z is a second long-chain structure. Therefore, it can have better safety to prevent the plasticizer from affecting the normal physiological functions by designing the plasticizer without a benzene ring structure. Further, the plasticizer can be prevented from affecting the normal physiological functions by designing the plasticizer structure that is dissimilar to estrogen.

According to the plasticizer of the present disclosure, in the formula (I), when a number of carbon atoms in X is XC, a number of carbon atoms in Y is YC, and a number of carbon atoms in Z is ZC, the following conditions can be

satisfied: 1 ≤ XC ≤ 6; 14 ≤ YC ≤ 25; and 14 ≤ ZC ≤ 25. Therefore, the plasticizer can be prevented from affecting the normal physiological functions by designing the plasticizer structure that is dissimilar to estrogen and without the benzene ring structure. Furthermore, the following conditions can be satisfied: 2 ≤ XC ≤ 5; 14 ≤ YC ≤ 24; and 14 ≤ ZC ≤ 24. Furthermore, the following conditions can be satisfied: 2 ≤ XC ≤ 4; 14 ≤ YC ≤ 23; and 14 ≤ ZC ≤ 23. Furthermore, the following conditions can be satisfied: 2 ≤ XC ≤ 3; 14 ≤ YC ≤ 22; and 14 ≤ ZC ≤ 22. Furthermore, the following conditions can be satisfied: 15 ≤ YC ≤ 21; and 15 ≤ ZC ≤ 21. Furthermore, the following conditions can be satisfied: 16 ≤ YC ≤ 20; and 16 ≤ ZC ≤ 20. Furthermore, the following conditions can be satisfied: 17 ≤ YC ≤ 19; and 17 ≤ ZC ≤ 19.

According to the plasticizer of the present disclosure, when a biodegradation rate on a 7th day is Dr7, the following condition of the biodegradation rate of the plasticizer on the 7th day is satisfied: 20% ≤ Dr7. Therefore, a short-term biodegradation rate of the plasticizer can be improved by satisfying the biodegradation rate of the plasticizer on the 7th day. Furthermore, the following condition can be satisfied: 30% ≤ Dr7. Furthermore, the following condition can be satisfied: 40% ≤ Dr7. Furthermore, the following condition can be satisfied: 50% ≤ Dr7. Furthermore, the following condition can be satisfied: 60% ≤ Dr7. Furthermore, the following condition can be satisfied: 65% ≤ Dr7 ≤ 100%.

According to the plastic composition of the present disclosure, a weight percentage of the plastic in the plastic composition is 85% to 99.99%, and a weight percentage of the plasticizer in the plastic composition is 0.01% to 15%. Therefore, the plastic composition has the excellent biodegradation property and the good plasticizing effect by a specific mixing ratio. Furthermore, the weight percentage of the plastic in the plastic composition can be 87.5% to 99.99%, and the weight percentage of the plasticizer in the plastic composition can be 0.01% to 12.5%. Furthermore, the weight percentage of the plastic in the plastic composition can be 90% to 99.99%, and the weight percentage of the plasticizer in the plastic composition can be 0.01 % to 10%. Furthermore, the weight percentage of the plastic in the plastic composition can be 92.5% to 99.99%, and the weight percentage of the plasticizer in the plastic composition can be 0.01% to 7.5%. Furthermore, the weight percentage of the plastic in the plastic composition can be 95% to 99.99%, and the weight percentage of the plasticizer in the plastic composition can be 0.01% to 5%.

According to the plastic composition of the present disclosure, when a biodegradation rate on a 14th day is Dr14, the following condition of the biodegradation rate of the plastic composition on the 14th day is satisfied: 1% ≤ Dr14. Therefore, an initial biodegradation rate of the plastic composition can be improved by satisfying the biodegradation rate of the plastic composition on the 14th day. Furthermore, the following condition can be satisfied: 2.5% ≤ Dr14. Furthermore, the following condition can be satisfied: 5% ≤ Dr14. Furthermore, the following condition can be satisfied: 7.5% ≤ Dr14. Furthermore, the following condition can be satisfied: 8% ≤ Dr14. Furthermore, the following condition can be satisfied: 9% ≤ Dr14 ≤ 100%.

According to the plastic composition of the present disclosure, when a biodegradation rate on a 21th day is Dr21, the following condition of the biodegradation rate of the plastic composition on the 21th day is satisfied: 6% ≤ Dr21. Therefore, an extreme short-term biodegradation rate of the plastic composition can be improved by satisfying the biodegradation rate of the plastic composition on the 21th day. Furthermore, the following condition can be satisfied: 10% ≤ Dr21. Furthermore, the following condition can be satisfied: 12.5% ≤ Dr21. Furthermore, the following condition can be satisfied: 15% ≤ Dr21. Furthermore, the following condition can be satisfied: 17.5% ≤ Dr21. Furthermore, the following condition can be satisfied: 20% ≤ Dr21. Furthermore, the following condition can be satisfied: 24% ≤ Dr21 ≤ 100%.

According to the plastic composition of the present disclosure, when a biodegradation rate on a 28th day is Dr28, the following condition of the biodegradation rate of the plastic composition on the 28th day is satisfied: 15% ≤ Dr28. Therefore, a short-term biodegradation rate of the plastic composition can be improved by satisfying the biodegradation rate of the plastic composition on the 28th day. Furthermore, the following condition can be satisfied: 20% ≤ Dr28. Furthermore, the following condition can be satisfied: 25% ≤ Dr28. Furthermore, the following condition can be satisfied: 30% ≤ Dr28. Furthermore, the following condition can be satisfied: 35% ≤ Dr28 ≤ 100%.

According to the plastic composition of the present disclosure, when a biodegradation rate on a 42th day is Dr42, the following condition of the biodegradation rate of the plastic composition on the 42th day is satisfied: 20% ≤ Dr42. Therefore, a mid-term biodegradation rate of the plastic composition can be improved by satisfying the biodegradation rate of the plastic composition on the 42th day. Furthermore, the following condition can be satisfied: 25% ≤ Dr42. Furthermore, the following condition can be satisfied: 30% ≤ Dr42. Furthermore, the following condition can be satisfied: 35% ≤ Dr42. Furthermore, the following condition can be satisfied: 40% ≤ Dr42. Furthermore, the following condition can be satisfied: 45% ≤ Dr42.

The plastic composition of the present disclosure further includes at least one non-biodegradable plastic. A weight percentage of the non-biodegradable plastic in the plastic composition is less than a weight percentage of the polylactic acid in the plastic composition. Therefore, the plastic composition including the non-biodegradable plastic is helpful for forming the plastic composition and improving the stability of the structure. Further, due to the weight percentage of the non-biodegradable plastic in the plastic composition is less than the weight percentage of the polylactic acid in the plastic composition, the plastic composition can be ensured to have a good biodegradation rate.

According to the plasticizer of the present disclosure, when a molecular weight of the plasticizer is Mw, the following condition is satisfied: 500 g/mole ≤ Mw. Therefore, the plasticizer is not easily absorbed by the human body

and can have the excellent safety by designing the plasticizer with the specific molecular weight. Furthermore, the following condition can be satisfied: 510 g/mole ≤ Mw ≤ 1000 g/mole. Furthermore, the following condition can be satisfied: 520 g/mole ≤ Mw ≤ 900 g/mole. Furthermore, the following condition can be satisfied: 530 g/mole ≤ Mw ≤ 800 g/mole. Furthermore, the following condition can be satisfied: 540 g/mole ≤ Mw ≤ 700 g/mole. Furthermore, the following condition can be satisfied: 550 g/mole ≤ Mw ≤ 600 g/mole. Furthermore, the following condition can be satisfied: 580 g/mole ≤ Mw ≤ 590 g/mole.

According to the plasticizer of the present disclosure, in the formula (I), each of X, Y and Z is a straight-chain structure. Therefore, due to the straight-chain molecule has a higher boiling point than the branched-chain molecule, the plasticizer can be prevented from being adsorbed by the human body after being vaporized by the high temperature.

According to the plasticizer of the present disclosure, in the formula (I), each of Y and Z has at least one double bond. Therefore, due to the structure of each of Y and Z has at least one double bond, the biodegradation rate of the plasticizer can be improved.

According to the plasticizer of the present disclosure, when a pyrolysis temperature of the plasticizer is Pt, the following condition is satisfied: 100°C ≤ Pt ≤ 300°C. Therefore, the high pyrolysis temperature of the plasticizer can help to stabilize the structure of the plasticizer at the high temperature. Furthermore, the following condition can be satisfied: 125°C ≤ Pt ≤ 300°C. Furthermore, the following condition can be satisfied: 150°C ≤ Pt ≤ 300°C. Furthermore, the following condition can be satisfied: 150°C ≤ Pt ≤ 280°C. Furthermore, the following condition can be satisfied: 180°C ≤ Pt ≤ 280°C. Furthermore, the following condition can be satisfied: 200°C ≤ Pt ≤ 280°C. Furthermore, the following condition can be satisfied: 200°C ≤ Pt ≤ 260°C.

According to the plasticizer of the present disclosure, when a biodegradation rate on a 21th day is Dr21, the following condition of the biodegradation rate of the plasticizer on the 21th day is satisfied: 65% ≤ Dr21. Therefore, a mid-term biodegradation rate of the plasticizer can be improved by satisfying the biodegradation rate of the plasticizer on the 21th day. Furthermore, the following condition can be satisfied: 70% ≤ Dr21. Furthermore, the following condition can be satisfied: 75% ≤ Dr21. Furthermore, the following condition can be satisfied: 80% ≤ Dr21. Furthermore, the following condition can be satisfied: 85% ≤ Dr21 ≤ 100%.

The plastic composition of the present disclosure can include the biodegradable plasticizer and the biodegradable plastic, and the non-biodegradable plasticizer and the non-biodegradable plastic are added as required to achieve the balance of function and biodegradable effect.

The plastic composition of the present disclosure can further include at least one additive, such as a stabilizer, a lubricant, a static coagulant, a flame retardant, a colorant, a filler, an antistatic agent, a light stabilizer, a foaming agent, a coupling agent and a flexibilizer. The stabilizer can be calcium stearate. The lubricant can be fatty acid. The flame retardant can be antimony trioxide. The colorant can be antimony dioxide. The filler can be a calcium carbonate, a glass fiber, an asbestos fiber, a carbon fiber, a boron fiber, a copper disulfide, a glimmer, an asbestos, a silica, a silicate, a metal oxide, a carbon black, a glass bead or a wood flour. The antistatic agent can be stearylaminopropyldimethyl-β-hydroxyethylamine nitrate. The light stabilizer can be salicylate, benzophenone, benzotriazole, substituted acryl, three or organic complex. The foaming agent can be azodicarbonamide. Therefore, the characteristic of the plastic, such as the impact resistance, the weather resistance, the heat resistance, the fluidity, the elasticity, the corrosion resistance, the deformation resistance, the light transmittance, the latent modification, the antistatic property, the photolysis resistance, the specific gravity or the toughness can be changed by modifying the plastic properties.

The biodegradable plastic in the plastic composition of the present disclosure can be polylactic acid (PLA), and also can be starch, poly(butyleneadipate-co-terephthalate) (PBAT), polybutylene succinate (PBS), poly propylene carbonate (PPC), polyhydroxyalkanoates (PHA), polycaprolactone (PCL), polyvinyl alcohol (PVA), polyglycolide (PGA), polyglycerol sebacate (PGS), polyhydroxybutyrate (PHB) or copolymers formed from the above polymer monomers.

The non-biodegradable plastic in the plastic composition of the present disclosure can be polyethylene terephthalate (PET), high-density polyethylene (HDPE), polyvinyl chloride (PVC), low-density polyethylene (LDPE), polypropene (PP), polystyrene (PS), polycarbonate (PC), polymethylmethacrylate (PMMA), acrylonitrile butadiene styrene (ABS), cyclic olefin copolymer (COC) or melamine resin or a mixture formed form the above plastic.

In the plasticizer of the present disclosure, the larger molecular weight means that the molecular weight of the plasticizer is greater than 500 g/mole, which is not limited by the plastic materials, and can plasticize different biodegradable plastics, so that has more wide applicability and convenience.

The plasticizer of the present disclosure can be the amide ester compound, and the amide ester compound refers to an amide compound having an ester group.

In the plasticizer of the present disclosure, in the formula (I), the structure of each of X, Y and Z has carbon atoms, and also can have at least one of sulfur atoms, oxygen atoms and nitrogen atoms. The structure of each of X, Y and Z has carbon-carbon single bonds, and also can have at least one of carbon-carbon double bonds, carbon-carbon triple bonds, carbon-oxygen bonds, carbon-nitrogen bonds, disulfide bonds, sulfur-oxygen bonds, sulfur-carbon bonds, sulfur-nitrogen bonds, double nitrogen bonds, nitrogen oxygen bonds and double oxygen bonds. The structure of each of X, Y and Z has an alkyl group, and can also have at least one of an alkenyl group, an alkynyl group, a hydroxyl group,

a carbonyl group, an aldehyde group, a carbonate group, a carboxyl group, an ether group, an ester group, an amide group, a primary amine group, a secondary amine group, a tertiary amine group, a ketimine group and an aldimine group. XC, YC and ZC can be 2, 17, 17, respectively, and then the plasticizer is 2-[(9Z)-9-octadecenylamino]ethyl(9Z)-9-octadecenoate. XC, YC and ZC can be 1, 14, 14, respectively, and then the plasticizer is 2-[(9Z)-9-pentadecenylamino] methyl(9Z)-9-pentadecenoate. XC, YC and ZC can be 6, 25, 25, respectively, and then the plasticizer is 2-[(9Z)-9-hexacosenylamino]hexyl(9Z)-9-hexacosenoate. The double bond position can also be on the 10th carbon of Y and Z at the same time, and then the plasticizer is 2-[(10Z)-10-hexacosenylamino]hexyl(10Z)-10-hexacosenoate.

The condition of the biodegradation rate in the present disclosure uses ASTM-D5338 as a standard method. The percentage of biodegradation rate is calculated by a unit of weight. The degradation product and the activated sludge (fertilizer) are mixed with each other in a weight ratio of 1:6, and the water content in the degradation tank is maintained at about 50% by perlite. The degradation tank is placed at 58 ± 2°C to observe for 180 days. The main components of the compost include livestock manure, soybean flour, bagasse, wood meal, wheat bran and mushroom-raising waste bag, and the components of the perlite are shown in Table 1.

Table 1

| Substance | $SiO_2$ | $Al_2O_3$ | $Fe_2O_3$ | CaO | $K_2O$ | $Na_2O$ | MgO | $H_2O$ |
|---|---|---|---|---|---|---|---|---|
| Content (%) | 68~74 | ~12 | 0.5~3.6 | 0.7~1.0 | 2~3 | 4~5 | 0.3 | 2.3~6.4 |

The fresh 0.1 M KOH aqueous solution is prepared during the testing and monitoring process, and dispensed into a 50 mL of PP tank. Then, the PP tank is placed in the degradation tank, and incubated in a temperate box (58 ± 2°C). The released $CO_2$ during the decomposition of the degradation product is absorbed by the KOH aqueous solution. The replacement frequency of KOH is 6 times/week at the initial stage of degradation (1-30 days), and changed to 2 times/week after one month until satisfied with 180 days. The total test time can also be greater than 180 days. The value of the collected KOH is measured with a pH meter and compared with the control group (0.1 M KOH aqueous solution is placed in the temperate box without degradation reaction), and then convert the hydrogen ion concentration. Then, the $CO_2$ weight is converted by the hydrogen ion concentration and estimated the biodegradation rate. The conversion formula mentioned above is as follows: hydroxyl concentration change = hydroxyl concentration in the control group - hydroxyl concentration in the experimental group = hydrogen ion concentration. Carbon dioxide weight (unit: mg) = hydrogen ion concentration × 44/2 × 50 mL (44 is the molecular weight of carbon dioxide, 2 is the carbon dioxide reaction equilibrium coefficient, and 50 mL is the volume of aqueous solution). Inorganization (decomposition) percentage = carbon dioxide weight change / theoretical carbon dioxide content for complete decomposition of the composition × 100%.

In the biodegradation rate for the nth day in the present disclosure, n can be the 7th day, the 8th day, the 9th, the 10th day, the 14th day, the 24th day, the 25th day, the 26th day, the 28th day, the 29th day, the 30th day, the 31st day, the 42nd day, the 52nd day, the 56th day, the 59th day, the 63rd day, the 66th day, the 70th day, the 73rd day, the 77th day, the 80th day, the 84th day, the 87th day, the 91st day, the 94th day, the 98th day, the 101st day, the 150th day, the 154th day, the 157th day, the 175th day, the 178th day, the 182nd day or the 185th day, or can be the biodegradation rate for any day from the 1st day to the 185th day.

Each technical feature in the abovementioned plastic composition and the plasticizer can be configured in combination to achieve the corresponding effect.

A plastic product includes the abovementioned plastic composition or the abovementioned plasticizer. Further, the plastic product can be a packaging film, a composite paper, a disposable container or a storage utensil, a disposable tableware, a food packaging, a binding tape, a rope, a wrapping film, an agricultural seedling cup and a plate, a mushroom space bag, a fertilizer bag, an agricultural tape or a belt, an agricultural tiling or a covering material, an agricultural plug tray, a vegetable and a fruit bagging, an agricultural border covering membrane, a surgical needle, a surgical membrane, a bone plate, a drug carrier, an inorganic mixture, a dental filling material, a biological suture anchor, a biological tissue scaffold, a bone nail, a biological tissue filler, a biological suture, a biological experimental petri dish, a biological experimental container, a biological experimental micropipette, a biological experimental centrifuge tube or an experimental glove, etc.

According to the abovementioned embodiments, the specific comparative examples and examples are provided below and described in detail with the drawings.

<1st comparative example>

The plastic composition of the 1st comparative example only includes the plastic, and the plastic contained in the plastic composition is polylactic acid (PLA). Please refer to Table 2A, which shows the components and the contents

of the plastic composition of the 1st comparative example.

| Table 2A | | |
|---|---|---|
| Plastic | Component | PLA |
| | Weight (g) | 100 |
| | Weight percentage (%) | 100 |
| | | |
| Plasticizer | Component | |
| | Weight (g) | 0 |
| | Weight percentage (%) | 0 |
| | | |
| Total weight (g) | | 100 |

Please refer to Fig. 1 and Table 2B, Fig. 1 is a graph between the biodegradation rate and the number of day of the plastic composition of the 1st comparative example. Table 2B shows the value of the biodegradation rate of the plastic composition of the 1st comparative example at different days.

| Table 2B | |
|---|---|
| Day | Biodegradation rate (%) |
| 1 | -0.7 |
| 2 | -0.7 |
| 3 | -0.7 |
| 4 | -0.7 |
| 5 | -0.7 |
| 7 | -1.1 |
| 8 | -0.9 |
| 9 | -1.1 |
| 10 | -1.1 |
| 11 | -1.3 |
| 12 | -1.3 |
| 13 | |
| 14 | -1.3 |
| 15 | -0.9 |
| 16 | -0.1 |
| 17 | 0.8 |
| 18 | 1.5 |
| 19 | 2.1 |
| 21 | 5.5 |
| 22 | 6.3 |
| 23 | 7.6 |
| 24 | 9.2 |
| 25 | 9.3 |

(continued)

| Table 2B | |
|---|---|
| Day | Biodegradation rate (%) |
| 26 | 9.2 |
| 28 | 10.7 |
| 29 | 10.3 |
| 30 | 11.3 |
| 31 | 11.3 |
| 35 | 13.6 |
| 38 | 15.9 |
| 42 | 19.1 |
| 45 | 21.5 |
| 49 | 24.5 |
| 52 | 27.0 |
| 56 | 30.3 |
| 59 | 33.0 |
| 63 | 36.1 |
| 66 | 38.4 |
| 70 | 41.9 |
| 73 | 43.7 |
| 77 | 46.2 |
| 80 | 48.6 |
| 84 | 50.8 |
| 87 | 52.1 |
| 91 | 53.8 |
| 94 | 54.7 |
| 98 | 55.8 |
| 101 | 56.4 |
| 105 | |
| 106 | 57.3 |
| 108 | 57.5 |
| 112 | 58.0 |
| 115 | 58.2 |
| 119 | 58.5 |
| 122 | 58.7 |
| 126 | |
| 129 | 58.8 |
| 133 | 58.8 |
| 136 | 58.8 |
| 140 | 58.6 |

(continued)

| Table 2B | |
|---|---|
| Day | Biodegradation rate (%) |
| 143 | 58.2 |
| 147 | 58.0 |
| 150 | 58.0 |
| 154 | 58.0 |
| 157 | 58.0 |
| 161 | 57.7 |
| 164 | 57.7 |
| 168 | 57.7 |
| 171 | 57.2 |
| 175 | 57.2 |
| 178 | 57.2 |
| 182 | 57.2 |
| 185 | 56.9 |

<2nd comparative example>

The plastic composition of the 2nd comparative example includes the plastic and the plasticizer, and the plastic contained in the plastic composition is poly(ethylene terephthalateco-1 ,4-cylclohexylenedimethylene terephthalate) (PETG). The plasticizer contained in the plastic composition is the amide ester compound, and the details of the plasticizer of the 2nd comparative example are shown in Table 3A.

| Table 3A | |
|---|---|
| Reactant A | Ethanolamine |
| Reactant B | Oleic acid |
| Product | (2-[(9Z)-octadec-9-enamido]ethyl(9Z)-octadec-9-enoate) |
| Structure | 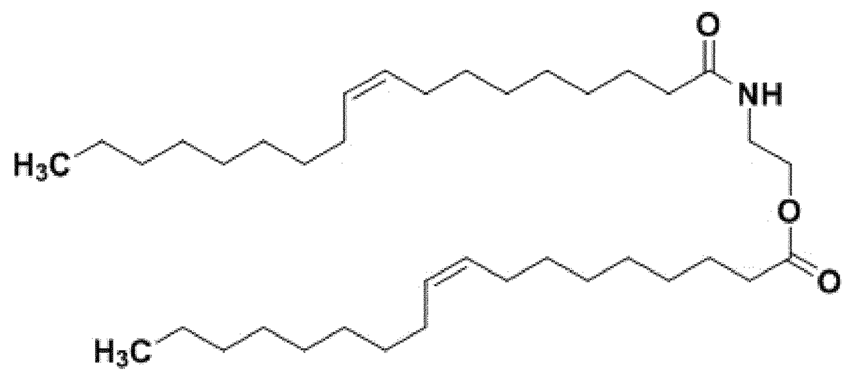 |
| SMILES Notation | CCCCCCCC\C=C/CCCCCCCC(=O)NCCOC(=O)CCCCCCC\C=C/CCCC CCCC |
| Structure schematic | 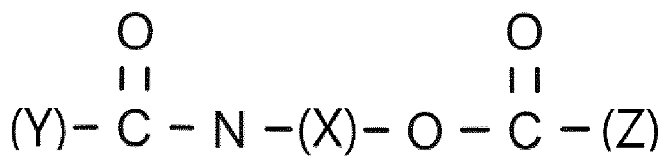 |

(continued)

| Table 3A | |
|---|---|
| XC | 2 |
| YC | 17 |
| ZC | 17 |
| Pt (°C) | 200~260 |
| Mw (g/mole) | 589 |

Furthermore, please refer to Table 3B, which shows the components and the contents of the plastic composition of the 2nd comparative example.

| Table 3B | | |
|---|---|---|
| Plastic | Component | PETG |
| | Weight (g) | 86 |
| | Weight percentage (%) | 86 |
| | | |
| Plasticizer | Component | amide ester compound |
| | Weight (g) | 14 |
| | Weight percentage (%) | 14 |
| | | |
| Total weight (g) | | 100 |

Please refer to Fig. 2 and Table 3C, Fig. 2 is a graph between the biodegradation rate and the number of day of the plastic composition of the 2nd comparative example. Table 3C shows the value of the biodegradation rate of the plastic composition of the 2nd comparative example at different days.

| Table 3C | |
|---|---|
| Day | Biodegradation rate (%) |
| 1 | 0.2 |
| 2 | 0.5 |
| 3 | 3.1 |
| 4 | 3.8 |
| 5 | 4.0 |
| 7 | 3.5 |
| 8 | 4.4 |
| 9 | 4.4 |
| 10 | 4.7 |
| 11 | 4.1 |
| 12 | 4.4 |
| 13 | |
| 14 | 4.7 |
| 15 | 4.2 |

(continued)

| Table 3C | |
|---|---|
| Day | Biodegradation rate (%) |
| 16 | 4.2 |
| 17 | 4.2 |
| 18 | 3.9 |
| 19 | 3.3 |
| 21 | 3.1 |
| 22 | 2.4 |
| 23 | 2.2 |
| 24 | 2.2 |
| 25 | 1.8 |
| 26 | 1.4 |
| 28 | 3.0 |
| 29 | 3.2 |
| 30 | 3.6 |
| 31 | 2.6 |
| 35 | 2.3 |
| 38 | 2.5 |
| 42 | 2.9 |
| 45 | 3.0 |
| 49 | 2.5 |
| 52 | 2.7 |
| 56 | 3.0 |
| 59 | 3.4 |
| 63 | 3.4 |
| 66 | 3.8 |
| 70 | 3.8 |
| 73 | 3.2 |
| 77 | 3.0 |
| 80 | 3.0 |
| 84 | 2.6 |
| 87 | 2.4 |
| 91 | 2.6 |
| 94 | 2.2 |
| 98 | 1.8 |
| 101 | 1.6 |
| 105 | |
| 106 | 1.2 |
| 108 | 1.2 |

(continued)

| Table 3C | |
|---|---|
| Day | Biodegradation rate (%) |
| 112 | 0.9 |
| 115 | 0.1 |
| 119 | -0.1 |
| 122 | -0.4 |
| 126 | |
| 129 | -1.2 |
| 133 | -1.9 |
| 136 | -0.7 |
| 140 | -1.4 |
| 143 | -2.8 |
| 147 | -3.3 |
| 150 | -3.6 |
| 154 | -4.5 |
| 157 | -5.1 |
| 161 | -5.8 |
| 164 | -5.8 |
| 168 | -6.1 |
| 171 | -7.2 |
| 175 | -8.2 |
| 178 | -9.0 |
| 182 | -9.7 |
| 185 | -10.0 |

<3rd comparative example>

The plastic composition of the 3rd comparative example only includes the plasticizer, and the plasticizer contained in the plastic composition is acetyl tributyl citrate. Please refer to Table 4A, which shows the components and the contents of the plastic composition of the 3rd comparative example.

| Table 4A | | |
|---|---|---|
| Plastic | Component | |
| | Weight (g) | 0 |
| | Weight percentage (%) | 0 |
| | | |
| Plasticizer | Component | acetyl tributyl citrate |
| | Weight (g) | 100 |
| | Weight percentage (%) | 100 |
| | | |
| Total weight (g) | | 100 |

Please refer to Fig. 3 and Table 4B, Fig. 3 is a graph between the biodegradation rate and the number of day of the plastic composition of the 3rd comparative example. Table 4B shows the value of the biodegradation rate of the plastic composition of the 3rd comparative example at different days.

Table 4B

| Day | Biodegradation rate (%) |
|---|---|
| 1 | -0.4 |
| 2 | 2.3 |
| 3 | 7.8 |
| 4 | 9.9 |
| 5 | 11.2 |
| 7 | 13.3 |
| 8 | 15.9 |
| 9 | 17.2 |
| 10 | 20.9 |
| 11 | 23.6 |
| 12 | 26.1 |
| 13 | |
| 14 | 34.8 |
| 15 | 36.9 |
| 16 | 41.8 |
| 17 | 46.5 |
| 18 | 50.7 |
| 19 | 55.0 |
| 21 | 62.1 |
| 22 | 64.5 |
| 23 | 68.0 |
| 24 | 72.1 |
| 25 | 71.7 |
| 26 | 74.7 |
| 28 | 81.8 |
| 29 | 83.7 |
| 30 | 84.7 |
| 31 | 85.1 |
| 35 | 87.1 |
| 38 | 88.0 |
| 42 | 88.8 |
| 45 | 89.3 |
| 49 | 89.7 |
| 52 | 90.0 |
| 56 | 90.9 |

(continued)

| Table 4B | |
|---|---|
| Day | Biodegradation rate (%) |
| 59 | 91.2 |
| 63 | 91.7 |
| 66 | 92.1 |
| 70 | 92.5 |
| 73 | 92.3 |
| 77 | 92.6 |
| 80 | 93.0 |
| 84 | 92.8 |
| 87 | 92.8 |
| 91 | 93.0 |
| 94 | 92.6 |
| 98 | 92.6 |
| 101 | 92.6 |
| 105 | |
| 106 | 92.6 |
| 108 | 92.6 |
| 112 | 92.4 |
| 115 | 91.8 |
| 119 | 91.8 |
| 122 | 91.8 |
| 126 | |
| 129 | 91.3 |
| 133 | 90.6 |
| 136 | 90.1 |
| 140 | 89.6 |
| 143 | 88.7 |
| 147 | 88.4 |
| 150 | 88.4 |
| 154 | 87.9 |
| 157 | 87.3 |
| 161 | 86.7 |
| 164 | 87.0 |
| 168 | 87.0 |
| 171 | 86.0 |
| 175 | 85.0 |
| 178 | 84.3 |
| 182 | 83.7 |

(continued)

| Table 4B | |
|---|---|
| Day | Biodegradation rate (%) |
| 185 | 83.3 |

<1st example>

The plastic composition of the 1st example includes the plastic and the plasticizer, the plastic contained in the plastic composition is polylactic acid, and the plasticizer contained in the plastic composition is the amide ester compound, wherein the details of the amide ester compound are shown in Table 3A, and will not be described herein. Please refer to Table 5A, which shows the components and the contents of the plastic composition of the 1st example.

| Table 5A | | |
|---|---|---|
| Plastic | Component | PLA |
| | Weight (g) | 95 |
| | Weight percentage (%) | 95 |
| | | |
| Plasticizer | Component | amide ester compound |
| | Weight (g) | 5 |
| | Weight percentage (%) | 5 |
| | | |
| Total weight (g) | | 100 |

Please refer to Fig. 4 and Table 5B, Fig. 4 is a graph between the biodegradation rate and the number of day of the plastic composition of the 1st example. Table 5B shows the value of the biodegradation rate of the plastic composition of the 1st example at different days.

| Table 5B | |
|---|---|
| Day | Biodegradation rate (%) |
| 1 | 0.5 |
| 2 | 2.0 |
| 3 | 2.4 |
| 4 | 2.4 |
| 5 | 2.4 |
| 7 | 1.7 |
| 8 | 3.3 |
| 9 | 3.1 |
| 10 | 3.9 |
| 11 | 5.0 |
| 12 | 5.8 |
| 13 | |
| 14 | 9.2 |
| 15 | 11.7 |

(continued)

| Table 5B | |
|---|---|
| Day | Biodegradation rate (%) |
| 16 | 14.2 |
| 17 | 15.9 |
| 18 | 17.2 |
| 19 | 18.7 |
| 21 | 24.8 |
| 22 | 28.8 |
| 23 | 32.1 |
| 24 | 32.4 |
| 25 | 33.2 |
| 26 | 33.8 |
| 28 | 38.4 |
| 29 | 39.4 |
| 30 | 40.9 |
| 31 | 41.0 |
| 35 | 46.3 |
| 38 | 51.1 |
| 42 | 55.6 |
| 45 | 57.4 |
| 49 | 59.0 |
| 52 | 60.1 |
| 56 | 61.0 |
| 59 | 61.7 |
| 63 | 62.1 |
| 66 | 62.7 |
| 70 | 62.8 |
| 73 | 62.5 |
| 77 | 62.5 |
| 80 | 62.8 |
| 84 | 62.5 |
| 87 | 62.4 |
| 91 | 62.4 |
| 94 | 62.0 |
| 98 | 61.9 |
| 101 | 61.5 |
| 105 | |
| 106 | 61.2 |
| 108 | 61.2 |

(continued)

| Table 5B | |
|---|---|
| Day | Biodegradation rate (%) |
| 112 | 61.0 |
| 115 | 60.3 |
| 119 | 60.2 |
| 122 | 60.2 |
| 126 | |
| 129 | 59.5 |
| 133 | 59.1 |
| 136 | 58.7 |
| 140 | 58.1 |
| 143 | 57.3 |
| 147 | 56.9 |
| 150 | 56.6 |
| 154 | 56.2 |
| 157 | 55.9 |
| 161 | 55.4 |
| 164 | 55.4 |
| 168 | 55.1 |
| 171 | 54.3 |
| 175 | 53.7 |
| 178 | 53.1 |
| 182 | 52.3 |
| 185 | 52.3 |

<2nd example>

The plastic composition of the 2nd example only includes the plasticizer, and the plasticizer contained in the plastic composition is the amide ester compound, wherein the details of the amide ester compound are shown in Table 3A, and will not be described herein. Please refer to Table 6A, which shows the components and the contents of the plastic composition of the 2nd example.

| Table 6A | | |
|---|---|---|
| Plastic | Component | |
| | Weight (g) | 0 |
| | Weight percentage (%) | 0 |
| | | |
| Plasticizer | Component | amide ester compound |
| | Weight (g) | 100 |
| | Weight percentage (%) | 100 |
| | | |

(continued)

| Table 6A | |
|---|---|
| Total weight (g) | 100 |

Please refer to Fig. 5 and Table 6B, Fig. 5 is a graph between the biodegradation rate and the number of day of the plastic composition of the 2nd example. Table 6B shows the value of the biodegradation rate of the plastic composition of the 2nd example at different days.

| Table 6B | |
|---|---|
| Day | Biodegradation rate (%) |
| 1 | 6.8 |
| 2 | 19.1 |
| 3 | 34.1 |
| 4 | 39.2 |
| 5 | 52.1 |
| 7 | 65.9 |
| 8 | 71.6 |
| 9 | 75.5 |
| 10 | 78.6 |
| 11 | 80.5 |
| 12 | 82.6 |
| 13 | 84.0 |
| 14 | |
| 15 | 84.6 |
| 16 | 85.1 |
| 17 | 85.9 |
| 18 | 86.0 |
| 19 | 87.3 |
| 21 | 88.1 |
| 22 | 88.5 |
| 23 | 88.5 |
| 24 | 88.8 |
| 25 | 89.0 |
| 26 | 89.3 |
| 28 | 89.7 |
| 29 | |
| 30 | |
| 31 | 89.9 |
| 35 | 90.5 |
| 38 | 90.9 |
| 42 | 91.4 |

(continued)

| Table 6B | |
|---|---|
| Day | Biodegradation rate (%) |
| 45 | 91.6 |
| 49 | 91.5 |
| 52 | 91.8 |
| 56 | 92.7 |
| 59 | 92.2 |
| 63 | 91.6 |
| 66 | 91.6 |
| 70 | 91.6 |
| 73 | 92.0 |
| 77 | 91.5 |
| 80 | 91.3 |
| 84 | 91.5 |
| 87 | 91.3 |
| 91 | 91.1 |
| 94 | 90.9 |
| 98 | 91.4 |
| 101 | 91.4 |
| 105 | 91.4 |
| 106 | |
| 108 | 91.0 |
| 112 | 91.4 |
| 115 | 92.1 |
| 119 | 91.9 |
| 122 | 91.9 |
| 126 | 92.2 |
| 129 | 92.6 |
| 133 | 92.7 |
| 136 | 92.2 |
| 140 | 92.2 |
| 143 | 92.2 |
| 147 | 92.2 |
| 150 | 92.3 |
| 154 | 92.5 |
| 157 | 92.5 |
| 161 | 92.1 |
| 164 | 91.8 |
| 168 | 91.5 |

(continued)

| Table 6B | |
|---|---|
| Day | Biodegradation rate (%) |
| 171 | 91.5 |
| 175 | 91.1 |
| 178 | 90.7 |
| 182 | 90.5 |
| 185 | |

<3rd example>

The plastic composition of the 3rd example includes the plastic and the plasticizer, the plastic contained in the plastic composition is polylactic acid, and the plasticizer contained in the plastic composition is the amide ester compound, wherein the details of the amide ester compound are shown in Table 3A, and will not be described herein. Please refer to Table 7, which shows the components and the contents of the plastic composition of the 3rd example.

| Table 7 | | |
|---|---|---|
| Plastic | Component | PLA |
| | Weight (g) | 96 |
| | Weight percentage (%) | 96 |
| | | |
| Plasticizer | Component | amide ester compound |
| | Weight (g) | 4 |
| | Weight percentage (%) | 4 |
| | | |
| Total weight (g) | | 100 |

<4th example>

The plastic composition of the 4th example includes the plastic and the plasticizer, the plastic contained in the plastic composition is polylactic acid, and the plasticizer contained in the plastic composition is the amide ester compound, wherein the details of the amide ester compound are shown in Table 3A, and will not be described herein. Please refer to Table 8, which shows the components and the contents of the plastic composition of the 4th example.

| Table 8 | | |
|---|---|---|
| Plastic | Component | PLA |
| | Weight (g) | 97 |
| | Weight percentage (%) | 97 |
| | | |
| Plasticizer | Component | amide ester compound |
| | Weight (g) | 3 |
| | Weight percentage (%) | 3 |
| | | |
| Total weight (g) | | 100 |

<5th example>

The plastic composition of the 5th example includes the plastic and the plasticizer, the plastic contained in the plastic composition is polylactic acid, and the plasticizer contained in the plastic composition is the amide ester compound, wherein the details of the amide ester compound are shown in Table 3A, and will not be described herein. Please refer to Table 9, which shows the components and the contents of the plastic composition of the 5th example.

| Table 9 | | |
|---|---|---|
| Plastic | Component | PLA |
| | Weight (g) | 98 |
| | Weight percentage (%) | 98 |
| | | |
| Plasticizer | Component | amide ester compound |
| | Weight (g) | 2 |
| | Weight percentage (%) | 2 |
| | | |
| Total weight (g) | | 100 |

<6th example>

The plastic composition of the 6th example includes the plastic and the plasticizer, the plastic contained in the plastic composition is polylactic acid, and the plasticizer contained in the plastic composition is the amide ester compound, wherein the details of the amide ester compound are shown in Table 3A, and will not be described herein. Please refer to Table 10, which shows the components and the contents of the plastic composition of the 6th example.

| Table 10 | | |
|---|---|---|
| Plastic | Component | PLA |
| | Weight (g) | 99 |
| | Weight percentage (%) | 99 |
| | | |
| Plasticizer | Component | amide ester compound |
| | Weight (g) | 1 |
| | Weight percentage (%) | 1 |
| | | |
| Total weight (g) | | 100 |

## Claims

1. A plastic composition, **characterized in** comprising:

   at least one plastic comprising a polylactic acid; and
   at least one plasticizer comprising an amide ester compound, wherein the amide ester compound has a biodegradability,
   wherein a weight percentage of the plastic in the plastic composition is 85% to 99.99%, and a weight percentage of the plasticizer in the plastic composition is 0.01% to 15%.

2. The plastic composition of claim 1, further comprising:

at least one non-biodegradable plastic, wherein a weight percentage of the at least one non-biodegradable plastic in the plastic composition is less than a weight percentage of the polylactic acid in the plastic composition.

**3.** The plastic composition of any one of claims 1 or 2, wherein the plasticizer has a structure represented by formula (I):

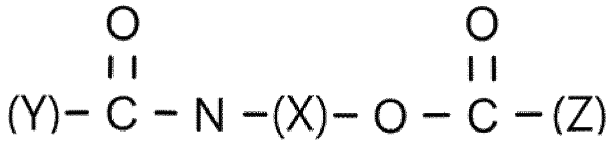

formula (I),

wherein X is a central structure, Y is a first long-chain structure, Z is a second long-chain structure, a number of carbon atoms in X is XC, a number of carbon atoms in Y is YC, a number of carbon atoms in Z is ZC, and the following conditions are satisfied:

$$1 \leq XC \leq 6;$$

$$14 \leq YC \leq 25;$$

and

$$14 \leq ZC \leq 25.$$

**4.** A plastic composition, **characterized in** comprising:

at least one plastic; and
at least one plasticizer, wherein the plasticizer has a biodegradability, and the plasticizer has a structure represented by formula (I):

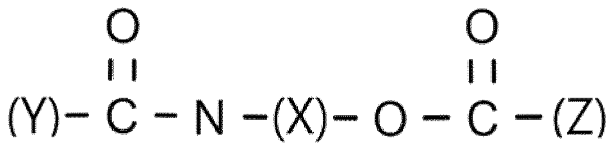

formula (I),

wherein X is a central structure, Y is a first long-chain structure, Z is a second long-chain structure, a number of carbon atoms in X is XC, a number of carbon atoms in Y is YC, a number of carbon atoms in Z is ZC, and the following conditions are satisfied:

$$1 \leq XC \leq 6;$$

$$14 \leq YC \leq 25;$$

and

$$14 \leq ZC \leq 25.$$

**5.** The plastic composition of any one of claims 1 to 4, wherein a molecular weight of the plasticizer is Mw, and the following condition is satisfied: 500 g/mole ≤ Mw.

**6.** The plastic composition of claims 4 or 5, wherein a weight percentage of the plastic in the plastic composition is 85% to 99.99%, and a weight percentage of the plasticizer in the plastic composition is 0.01% to 15%.

**7.** The plastic composition of any one of claims 3 to 6, wherein each of X, Y and Z is a straight-chain structure.

**8.** The plastic composition of any one of claims 3 to 7, wherein each of Y and Z has at least one double bond.

**9.** The plastic composition of any one of claims 1 to 8, wherein a pyrolysis temperature of the plasticizer is Pt, and the following condition is satisfied: 100°C ≤ Pt ≤ 300°C.

**10.** The plastic composition of any one of claims 1 to 9, wherein a biodegradation rate on a 14th day is Dr14, and the following condition of the biodegradation rate of the plastic composition on the 14th day is satisfied: 1% ≤ Dr14.

**11.** The plastic composition of any one of claims 1 to 10, wherein a biodegradation rate on a 21th day is Dr21, and the following condition of the biodegradation rate of the plastic composition on the 21th day is satisfied: 6% ≤ Dr21.

**12.** The plastic composition of any one of claims 1 to 11, wherein a biodegradation rate on a 28th day is Dr28, and the following condition of the biodegradation rate of the plastic composition on the 28th day is satisfied: 15% ≤ Dr28.

**13.** The plastic composition of any one of claims 1 to 12, wherein a biodegradation rate on a 42th day is Dr42, and the following condition of the biodegradation rate of the plastic composition on the 42th day is satisfied: 20% ≤ Dr42.

**14.** A plastic product, **characterized in** comprising:

the plastic composition of any one of claims 1 to 13.

**15.** A plasticizer, **characterized in** having a biodegradability, and the plasticizer having a structure represented by formula (I):

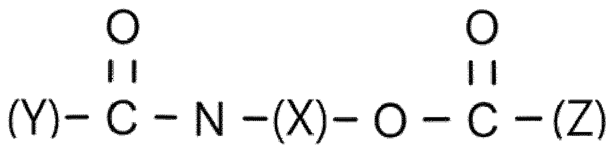

formula (I),

wherein X is a central structure, Y is a first long-chain structure, Z is a second long-chain structure, a number of carbon atoms in X is XC, a number of carbon atoms in Y is YC, a number of carbon atoms in Z is ZC, and the following conditions are satisfied:

$$1 \leq XC \leq 6;$$

$$14 \leq YC \leq 25;$$

and

$$14 \leq ZC \leq 25,$$

wherein a biodegradation rate on a 7th day is Dr7, and the following condition of the biodegradation rate of the plasticizer on the 7th day is satisfied: 20% ≤ Dr7.

**16.** The plasticizer of claim 15, wherein a molecular weight of the plasticizer is Mw, and the following condition is satisfied: 500 g/mole ≤ Mw.

**17.** The plasticizer of claims 15 or 16, wherein a biodegradation rate on a 21th day is Dr21, and the following condition

of the biodegradation rate of the plasticizer on the 21th day is satisfied: 65% $\leq$ Dr21.

**18.** The plasticizer of any one of claims 15 to 17, wherein each of X, Y and Z is a straight-chain structure.

**19.** The plasticizer of any one of claims 15 to 18, wherein each of Y and Z has at least one double bond.

**20.** The plasticizer of any one of claims 15 to 19, wherein a pyrolysis temperature of the plasticizer is Pt, and the following condition is satisfied: 100°C $\leq$ Pt $\leq$ 300°C.

**21.** A plastic product, **characterized in** comprising:
the plasticizer of any one of claims 15 to 20.

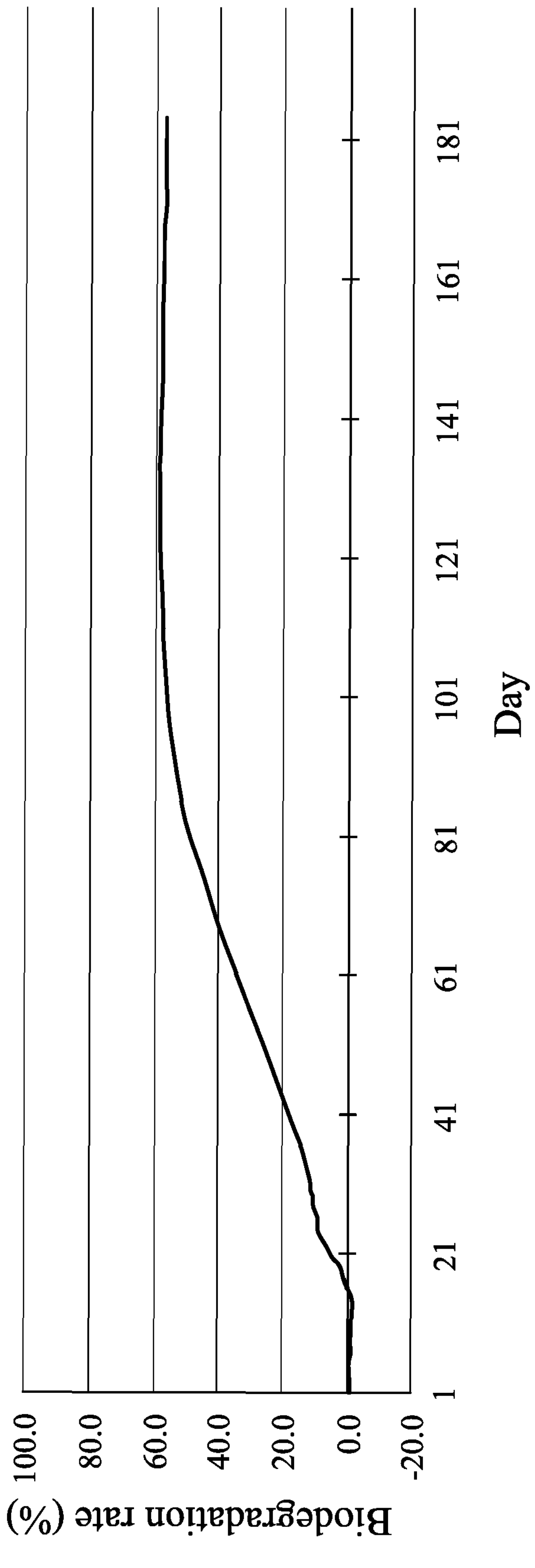
1st comparative example
Biodegradation rate (%)
100.0
80.0
60.0
40.0
20.0
0.0
-20.0
1
21
41
61
81
101
121
141
161
181
Day

Fig. 1

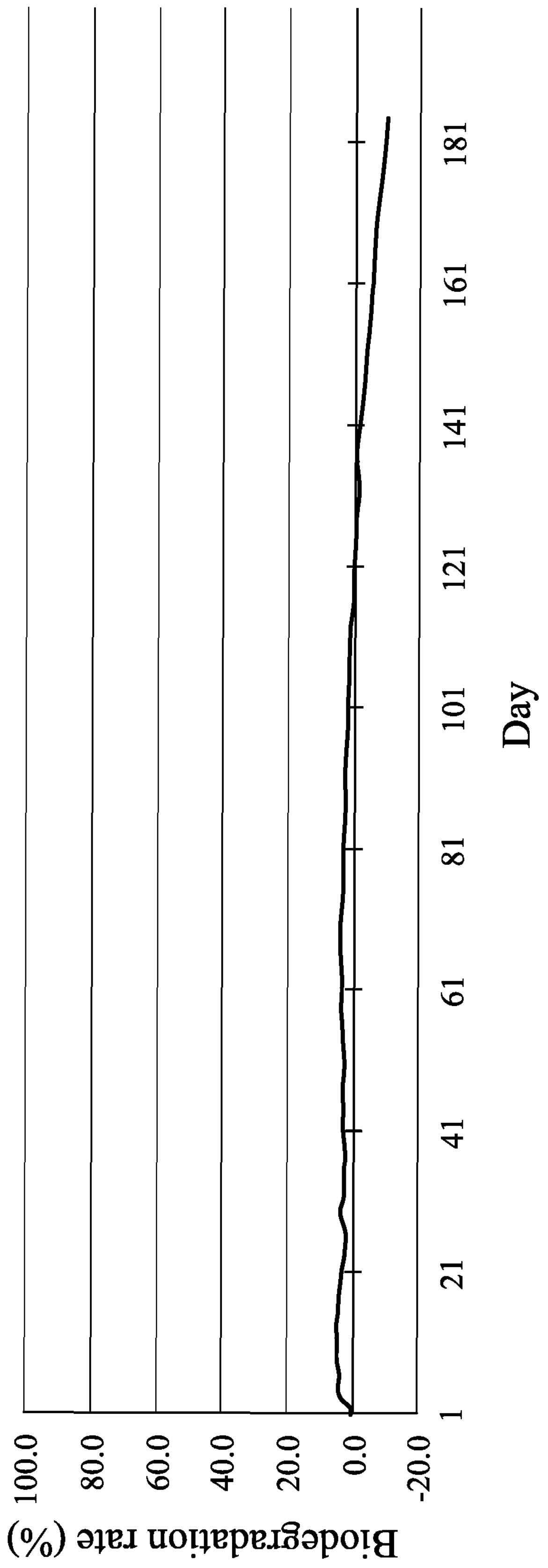
2nd comparative example
Biodegradation rate (%)
100.0
80.0
60.0
40.0
20.0
0.0
-20.0
1
21
41
61
81
101
121
141
161
181
Day

Fig. 2

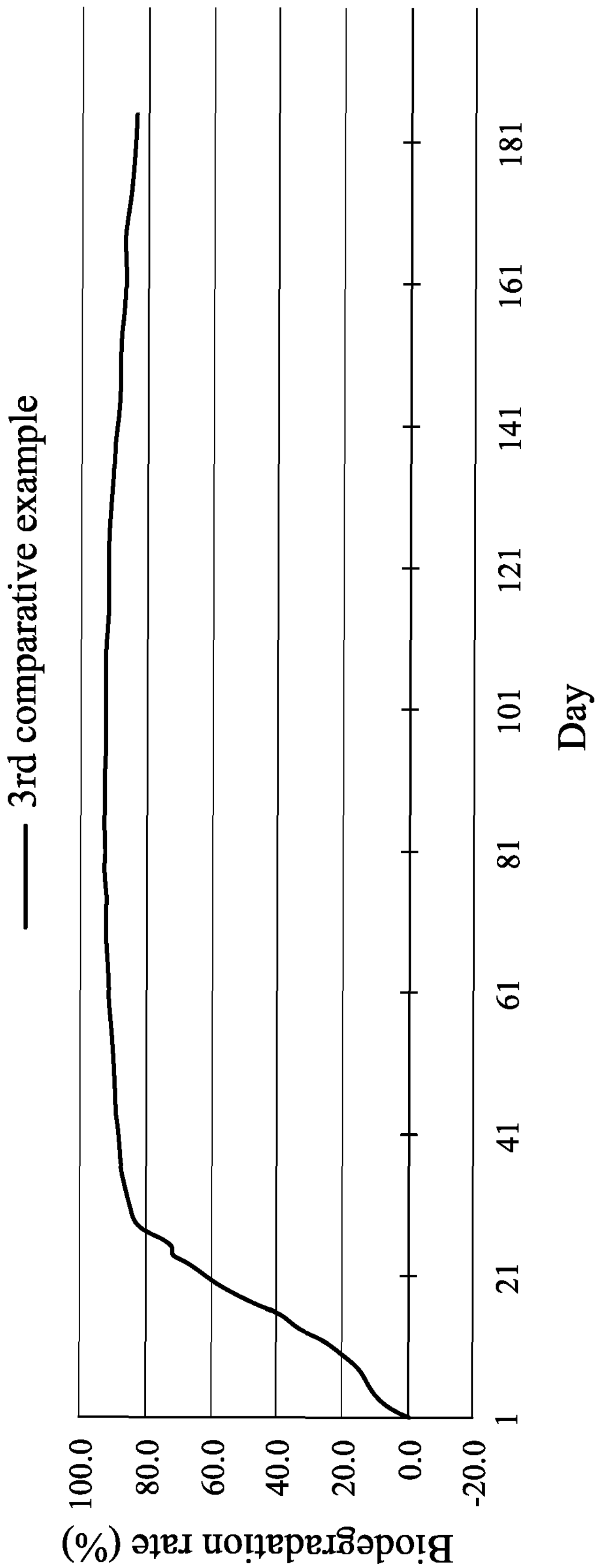
3rd comparative example
Biodegradation rate (%)
100.0
80.0
60.0
40.0
20.0
0.0
-20.0
1
21
41
61
81
101
121
141
161
181
Day

Fig. 3

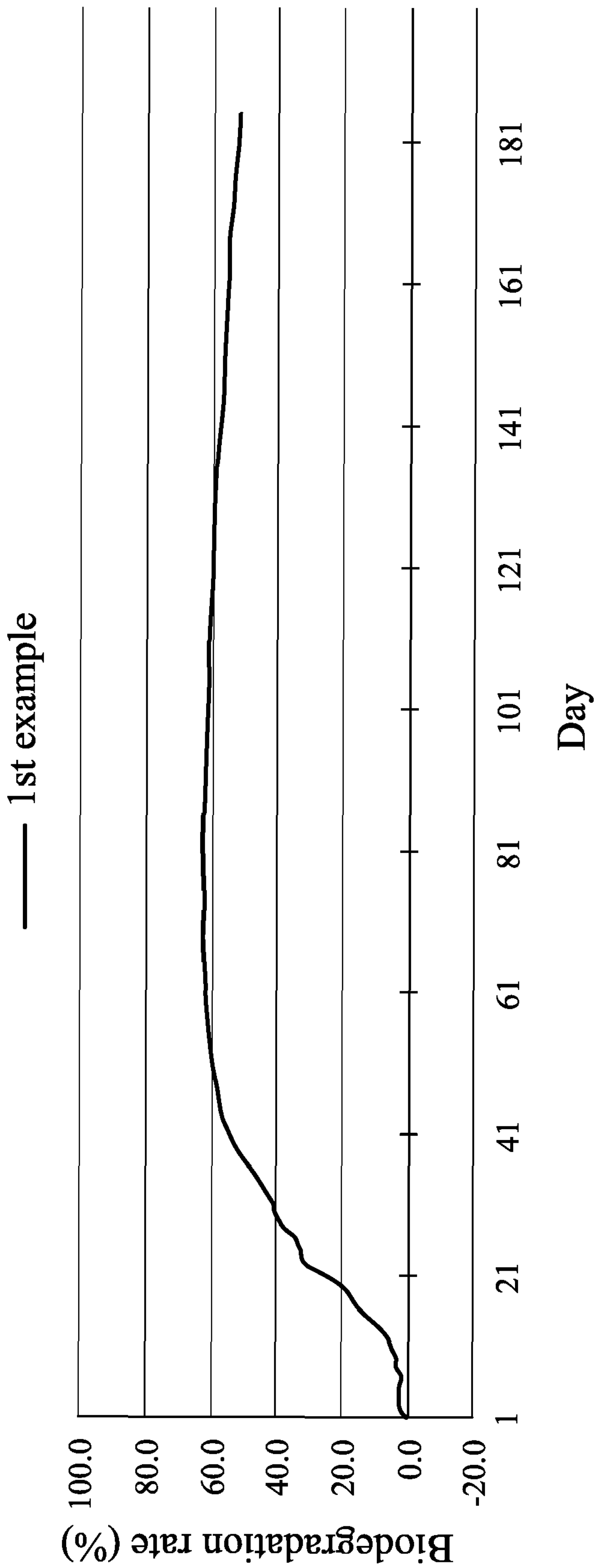
1st example
Biodegradation rate (%)
100.0
80.0
60.0
40.0
20.0
0.0
-20.0
1
21
41
61
81
101
121
141
161
181
Day

Fig. 4

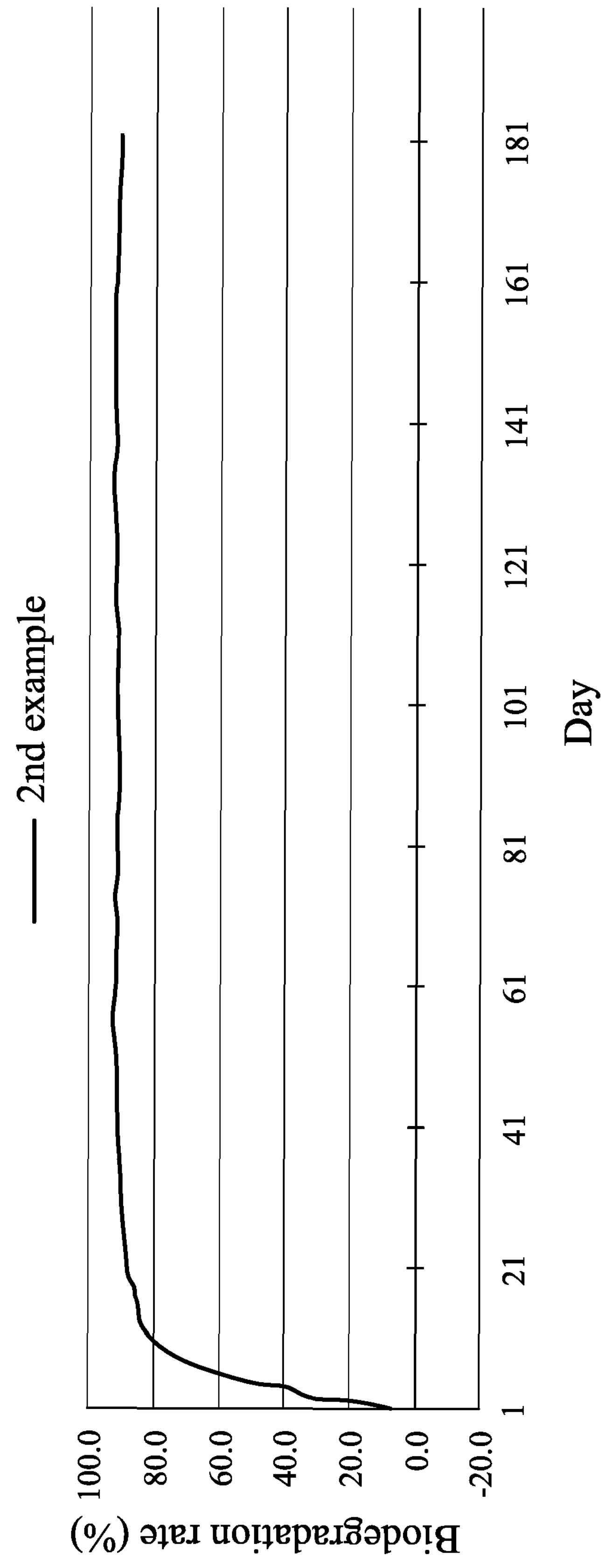
2nd example
Biodegradation rate (%)
100.0
80.0
60.0
40.0
20.0
0.0
-20.0
1
21
41
61
81
101
121
141
161
181
Day

Fig. 5

Europäisches Patentamt
European Patent Office
Office européen des brevets

# EUROPEAN SEARCH REPORT

Application Number
EP 23 16 7632

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DATABASE WPI<br>Week 201578<br>Thomson Scientific, London, GB;<br>AN 2015-680514<br>XP002809995,<br>-& JP 2015 193750 A (OSAKA GAS CHEM KK)<br>5 November 2015 (2015-11-05)<br>* abstract *<br>* paragraph [0056] - paragraph [0059] *<br>* examples 1-6 *<br>* claims 1-5,7 *<br>----- | 1,2,9-14 | INV.<br>C08K5/20 |
| A | EP 2 583 994 A1 (TORAY INDUSTRIES [JP])<br>24 April 2013 (2013-04-24)<br>* paragraph [0093] *<br>* examples 1-18; tables 1,2 *<br>* claims 1,3,5 *<br>----- | 1,2,9-14 | |
| X | TKACHEVA ANASTASIA ET AL:<br>"Pharmaceuticals and Surfactants from Alga-Derived Feedstock: Amidation of Fatty Acids and Their Derivatives with Amino Alcohols",<br>CHEMSUSCHEM,<br>vol. 8, no. 16,<br>24 August 2015 (2015-08-24), pages 2670-2680, XP093075164,<br>DE<br>ISSN: 1864-5631, DOI:<br>10.1002/cssc.201500526<br>* page 2671; figure 1; compound 5 *<br>----- | 15-20 | TECHNICAL FIELDS SEARCHED (IPC)<br>C08K |
| A | US 2010/093888 A1 (ENDO KOHEI [JP] ET AL)<br>15 April 2010 (2010-04-15)<br>* paragraph [0217] *<br>----- | 1-21 | |

The present search report has been drawn up for all claims

1

| Place of search | Date of completion of the search | Examiner |
|---|---|---|
| Munich | 22 August 2023 | Russell, Graham |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO.

EP 23 16 7632

This annex lists the patent family members relating to the patent documents cited in the above-mentioned European search report. The members are as contained in the European Patent Office EDP file on
The European Patent Office is in no way liable for these particulars which are merely given for the purpose of information.

22-08-2023

| Patent document cited in search report | Publication date | Patent family member(s) | Publication date |
|---|---|---|---|
| JP 2015193750 A | 05-11-2015 | JP 6556427 B2 | 07-08-2019 |
| | | JP 2015193750 A | 05-11-2015 |
| EP 2583994 A1 | 24-04-2013 | CN 102892817 A | 23-01-2013 |
| | | EP 2583994 A1 | 24-04-2013 |
| | | JP 5867084 B2 | 24-02-2016 |
| | | JP WO2011162046 A1 | 19-08-2013 |
| | | KR 20130089159 A | 09-08-2013 |
| | | WO 2011162046 A1 | 29-12-2011 |
| US 2010093888 A1 | 15-04-2010 | BR PI0807936 A2 | 01-07-2014 |
| | | CN 101622310 A | 06-01-2010 |
| | | EP 2116576 A1 | 11-11-2009 |
| | | HK 1136593 A1 | 02-07-2010 |
| | | JP 5525811 B2 | 18-06-2014 |
| | | JP WO2008102919 A1 | 03-06-2010 |
| | | KR 20090118938 A | 18-11-2009 |
| | | TW 200911914 A | 16-03-2009 |
| | | US 2010093888 A1 | 15-04-2010 |
| | | WO 2008102919 A1 | 28-08-2008 |

EPO FORM P0459

For more details about this annex : see Official Journal of the European Patent Office, No. 12/82